(19) Europäisches Patentamt
European Patent Office
Office européen des brevets

(11) EP 0 636 361 B1

(12) **FASCICULE DE BREVET EUROPEEN**

(45) Date de publication et mention de la délivrance du brevet:
**15.04.1998 Bulletin 1998/16**

(51) Int. Cl.[6]: **A61K 7/48**, A61K 7/06, C08G 77/42

(21) Numéro de dépôt: **94401599.9**

(22) Date de dépôt: **11.07.1994**

(54) **Compositions cosmétiques et utilisations**

Kosmetische Mittel und Verwendungen

Cosmetic compositions and uses

(84) Etats contractants désignés:
**CH DE ES FR GB IT LI NL**

(30) Priorité: **28.07.1993 FR 9309286**

(43) Date de publication de la demande:
**01.02.1995 Bulletin 1995/05**

(73) Titulaire: **L'OREAL**
**75008 Paris (FR)**

(72) Inventeurs:
- **Mougin, Nathalie**
  **F-75010 Paris (FR)**
- **Mondet, Jean**
  **F-93700 Drancy (FR)**

(74) Mandataire: **Dodin, Catherine**
**L'Oreal-D.P.I.,**
**90, rue du Général Roguet**
**92583 Clichy Cédex (FR)**

(56) Documents cités:
**WO-A-91/17215**

- **DIE MAKROMOLEKULARE CHEMIE, vol.187, no.6, Avril 1986, BASEL, CH pages 1371 - 1380 KOSSMEHL ET AL. 'hydrophilic silicone rubber materials containing dimethylsiloxane and urethane moieties in the main chain'**
- **POLYMER JOURNAL, vol.24, no.4, 15 Avril 1992, TOKYO, JP pages 347 - 355 OTSUKI ET AL. 'synthesis and properties of new poly(dimethylsiloxane)-urea-polyamide multiblock copolymers by diisocyanate method'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.1/3.4

## Description

La présente invention concerne de nouvelles compositions cosmétiques et certaines de leurs applications particulières.

Plus précisément encore, elle a pour objet de nouvelles compositions cosmétiques à propriétés filmogènes contenant des pseudo-latex particuliers, ainsi que diverses de leurs utilisations possibles, notamment dans le domaine des traitements cosmétiques (i.e. par voie topique) de la peau, des cheveux, des ongles et autres matières kératiniques.

Il est d'usage courant de mettre en oeuvre dans des formulations cosmétiques, en particulier dans des produits capillaires (shampooings, après-shampooings, lotions ou gels coiffants ou traitants, laques ou lotions de mise en forme, de mise en plis ou de fixation, etc.) ou dans des produits de maquillage (tels que par exemple vernis à ongle, mascaras, eye-liners, et autres), une proportion variable, selon la nature et la destination de la formulation, d'au moins une substance filmogène permettant de, ou visant à, conférer au support sur lequel elle est appliquée (c'est à dire, ici, l'une des parties superficielles du corps, telle que cheveux, cils, poils, peau, ongles,...) certaines caractéristiques améliorées.

Ainsi, par exemple, dans le cas particulier du traitement d'une chevelure, on recherche avant tout par cette technique plus de tenue et plus de douceur pour les cheveux, alors que dans le cas plus particulier des ongles, on recherche principalement l'obtention d'un film protecteur brillant et dur, adhérant parfaitement sur ces derniers.

Ces dernières années, un intérêt tout particulier s'est manifesté pour la réalisation de compositions cosmétiques filmogènes de type aqueux, et ceci dans le but de remplacer, pour des raisons notamment de sécurité et d'environnement, les habituelles substances filmogènes se présentant dans des supports organiques (en particulier des alcools).

Ainsi, il est maintenant connu d'utiliser dans certaines compositions cosmétiques des latex (c'est à dire des dispersions colloïdales aqueuses de particules de polymères) contenant, comme résines filmogènes, des polyuréthannes ou des polymères acryliques.

A titre d'exemples, il a été décrit dans la demande de brevet EP-A- 418 469, des compositions de vernis à ongles contenant des dispersions aqueuses de polyuréthannes aliphatiques et, dans la demande de brevet EP-A- 391 322, des vernis à ongles contenant une dispersion aqueuse d'un polyuréthanne et/ou d'un polyuréthanne copolymère.

Une résine filmogène, pour être satisfaisante dans des applications cosmétiques, se doit de présenter certaines caractéristiques ou propriétés contraignantes, parmi lesquelles on peut plus particulièrement citer, et ceci de manière non limitative, d'abord une très bonne affinité/compatibilité/innocuité vis-à-vis des différentes matières kératiniques (peau, cheveux et autres), ensuite de bonnes propriétés filmogènes par rapport à ces dernières (qualité et régularité du film déposé), et enfin de bonnes propriétés de rémanence (adhésivité, solidité), c'est à dire qu'elle doit être difficilement éliminable de son support par de simples lavages à l'eau ou à l'aide de détergents (shampooings) par exemple. Dans le cas des vernis à ongles, le film doit par ailleurs posséder une bonne résistance à l'abrasion mécanique. D'une manière générale, on notera qu'il est souvent difficile, dans la pratique, de trouver une substance filmogène susceptible de convenir effectivement à plusieurs, ou à toutes, les différentes applications cosmétiquement envisageables pour cette dernière (problème du compromis acceptable).

A certains égards, les substances filmogènes connues à ce jour, et en particulier celles mentionnées ci-avant, conviennent mal pour l'obtention de compositions présentant de bonnes propriétés cosmétiques, en raison notamment d'un manque notable de rémanence, en particulier de résistance à l'eau.

Un autre problème réside dans le fait que les films ainsi obtenus, en particulier dans le cadre des applications de type capillaires ou mascaras, présentent une brillance insuffisante. Cette brillance n'est en outre que faiblement rémanente, c'est à dire qu'elle disparaît rapidement sous l'action d'agents extérieurs (forte sensibilité à l'eau notamment).

Or, la brillance, ainsi que la rémanence de cette brillance, constitue aujourd'hui une propriété particulièrement recherchée dans le domaine de la cosmétique.

On voit donc qu'il existe actuellement dans l'état de l'art un fort besoin quant à pouvoir disposer de compositions filmogènes cumulant, et ceci pour un domaine varié d'applications possibles (cheveux, cils, peau, ongles,...), tous les avantages généralement recherchés ou désirables en cosmétique, à savoir notamment l'innocuité vis à vis des matières kératiniques, la facilité d'application et de mise en oeuvre, l'obtention de dépôts protecteurs fins et réguliers, la rémanence des propriétés adhésives, l'apport et la rémanence des propriétés de brillance, l'apport de douceur et de lubrification, de rigidité et de résistance à l'abrasion.

La présente invention vise justement à la satisfaction d'un tel besoin.

Ainsi, à la suite d'importantes recherches menées sur la question, il a été trouvé par la demanderesse, et ceci de façon inattendue et surprenante, qu'il était possible d'obtenir des compositions cosmétiques filmogènes convenant à de nombreuses applications et présentant d'excellentes propriétés, en particulier telles que celles ci-dessus énumérées, en utilisant des pseudo-latex bien particuliers. Cette découverte est à la base de la présente invention.

Conformément à la présente invention, il est donc maintenant proposé de nouvelles compositions cosmétiques caractérisées par le fait qu'elles comprennent, dans un support cosmétiquement acceptable, au moins un pseudo-latex à base d'un polycondensat multiséquencé dont la chaîne est constituée par la répétition d'au moins une séquence polysiloxane et d'au moins une séquence polyuréthanne et/ou polyurée, ladite séquence polyuréthanne et/ou polyurée

comportant en outre des groupements anioniques ou cationiques.

On entend désigner selon l'invention, et ceci en conformité avec l'acception commune, par l'expression "pseudo-latex" une suspension aqueuse stable constituée de fines particules, généralement sphériques, du polycondensat polysiloxanes/polyuréthannes tel que ci-dessus défini, ces particules ayant été obtenues par mise en dispersion, dans une phase aqueuse appropriée, dudit polycondensat à l'état déjà synthétisé.
L'expression "pseudo-latex" ne doit donc pas être confondue avec l'expression "latex" ou "latex synthétique" qui est certes également une suspension aqueuse constituée de particules d'un polymère ou d'un polycondensat, mais dont lesdites particules ont été classiquement obtenues directement par polymérisation (respectivement par polycondensation) en émulsion d'un ou plusieurs monomères dans une phase aqueuse appropriée.
En particulier, la synthèse d'un "latex" nécessite obligatoirement l'emploi d'agents tensioactifs, lesquels se retrouvent alors toujours dans la suspension finale.
A l'inverse, compte tenu de la nature ionique des polycondensats mis en oeuvre dans le cadre de l'invention, on s'affranchit d'un tel emploi. Ce point important sera repris plus en détails par la suite.

Pour une bonne compréhension de l'exposé qui va suivre, et en particulier de la définition des formules données, on commencera par expliquer, mais seulement dans ses grandes lignes directrices, le procédé général de synthèse des pseudo-latex utilisés dans le cadre de la présente invention.
Les détails du procédé seront donnés plus loin. De même, seront précisées ultérieurement les significations et valeurs de certains radicaux (R, B,...) et autres paramètres, qui n'apparaîtront donc dans la partie introductive donnée maintenant qu'à titre purement symbolique et de commodité.

Comme indiqué précédemment, cette synthèse passe donc tout d'abord par la préparation préalable du polycondensat destiné à être mis en suspension.
Ce polycondensat est essentiellement préparé selon un procédé en deux étapes. La première étape consiste en une réaction classique de polycondensation entre

(i) un polymère polysiloxane (ou silicone) présentant une fonction hydroxy ou une fonction amine aux extrémités de sa chaîne (i.e un $\alpha,\omega$-dihydroxypolysiloxane, ou un $\alpha,\omega$-diaminopolysiloxane, ou un $\alpha,\omega$-aminohydroxy- ou hydroxyaminopolysiloxane), et
(ii) un diisocyanate (présent en quantité stoechiométrique, ou en excès stoechiométrique, c'est à dire à plus de 2 moles par mole de silicone) ce par quoi l'on obtient une nouvelle silicone présentant cette fois une fonction isocyanate à chacune de ses extrémités de chaîne.

Dans une deuxième étape, on couple les chaînes du polycondensat obtenu précédemment au moyen d'un agent coupleur (en quantité variable choisie en fonction de la longueur de chaîne finale désirée) choisi parmi les diols et/ou les diamines et/ou les alcoolamines, de manière à obtenir finalement un nouveau polycondensat de plus longue chaîne.

Les réactions mises en oeuvre dans la première étape conduisent à un polysiloxane présentant à ses extrémités de chaîne, outre les fonctions isocyanates mentionnées ci-dessus, des motifs uréthanne et/ou urée, et ceci selon les mécanismes classiques d'une réaction de condensation opérée entre (i) une fonction isocyanate, telle que portée par le diisocyanate de départ, et (ii) une fonction alcool (création dans ce cas d'un motif uréthanne) ou d'une fonction amine (formation dans ce cas d'une fonction urée), telles que portées par le polysiloxane de départ, à savoir :

-(polysiloxane)-OH + O=C=N-R-N=C=O -----------> -(polysiloxane)-O-C(O)-NH-R-N=C=O
(diisocyanate)

et/ou

$$\text{-(polysiloxane)-}NH_2 + O{=}C{=}N\text{-}R\text{-}N{=}C{=}O \rightarrow \text{-(polysiloxane)-}NH\text{-}C(O)\text{-}NH\text{-}R\text{-}N{=}C{=}O$$

Les polycondensats obtenus à l'issue de cette première étape peuvent donc être en fait définis par la formule générale (1) suivante :

$$O{=}C{=}N\text{-}R\text{-}NH\text{-}C(O)\text{-}X^1\text{-(polysiloxane)-}X^1\text{-}C(O)\text{-}NH\text{-}R\text{-}N{=}C{=}O \quad (1)$$

dans laquelle $X^1$ peut donc représenter, séparément ou conjointement, -O- ou -NH-.

Dans la deuxième étape, les fonctions alcools et/ou amines de l'agent coupleur (agent coupleur que l'on peut ici symboliser de manière commode par OH-B-OH, ou $NH_2$-B-$NH_2$ ou bien encore $NH_2$-B-OH) viennent alors réagir, et ceci selon les mêmes mécanismes que ceux exposés pour la première étape, soit avec les fonctions isocyanates portées en bout de chaîne par le polycondensat polysiloxane de formule (1) ci-dessus, soit avec des fonctions isocyanates portées par du diisocyanate libre, lorsque ce dernier a été introduit en excès stoechiométrique lors de la première étape, donnant ainsi naissance dans la chaîne (plus longue) du nouveau polycondensat obtenu à une succession de motifs uréthanne et/ou urée, c'est à dire à des séquences de type polyuréthanne et/ou polyurée symbolisables par la formule (2) :

$$-\!\left( X^2 - B - X^2 - \underset{\underset{O}{\|}}{C} - NH - R - NH - \underset{\underset{O}{\|}}{C} - \right)_x \qquad (2)$$

dans laquelle $X^2$ représente -O- ou -NH-, et x est une valeur correspondant substantiellement au nombre de moles d'agent coupleur mises en oeuvre dans la réaction.

Comme indiqué précédemment, on obtient donc ainsi finalement un polycondensat constitué par la répétition de séquences polysiloxanes (correspondant simplement au polysiloxane d'origine, et tel qu'il apparaît dans la formule (1)) et de séquences polyuréthannes et/ou polyurées (formule (2)).

Selon un aspect extrêmement important de l'invention, les agents coupleurs (c'est à dire en fait le radical B) portent des groupements chimiquement anionisables ou cationisables, c'est à dire des groupements qui, et respectivement, lorsqu'ils sont soumis à l'action d'une base, donnent des groupement anioniques (c'est le cas par exemple des groupements carboxyliques) et lorsqu'ils sont soumis à l'action d'un acide, donnent des groupements cationiques (cas par exemple d'une amine tertiaire). La neutralisation des groupements anionisables (respectivement cationisables) par la base (respectivement par l'acide) peut alors être, au choix, soit partielle soit totale, selon les quantité d'agents neutralisants mises en oeuvre.

Le caractère ionisable (et ionisé après neutralisation) du polycondensat permet ainsi de s'affranchir de l'emploi d'agents tensioactifs lors de la préparation des pseudo-latex correspondants (autodispersabilité ). Ces pseudo-latex sont obtenus selon les méthodes classiques et connues de préparation des pseudo-latex, sous réserves toutefois de certaines particularités qui seront mentionnées plus en détails par la suite. En particulier, on peut souligner à nouveau que les pseudo-latex conformes à l'invention présentent un caractère ionique plus ou moins marqué.

Mais d'autres caractéristiques, aspects et avantages de l'invention apparaîtront maintenant de manière plus claire à la lecture de la description détaillée et complète qui va suivre, ainsi que des divers exemples concrets, mais nullement limitatifs, destinés à l'illustrer.

Comme indiqué précédemment, la chaîne du polycondensat ionique rentrant dans la composition des pseudo-latex utilisés dans le cadre de la présente invention est essentiellement caractérisée par le fait qu'elle est constituée par la répétition (ou alternance) de séquences de type polysiloxane et de séquences de type polyuréthanne et/ou polyurée, lesdites séquences polyuréthannes et/ou polyurées comportant des groupement ioniques de type anioniques ou cationiques.

La répétition des séquences ci-dessus peut être de type aléatoire, mais elle est de préférence de type régulièrement alternée. En outre, le rapport en nombre entre les séquences de type polyuréthanne et/ou polyurée et les séquences de type polysiloxane est généralement compris entre 1 et 10, de préférence entre 1 et 3.

Les poids moléculaires des polycondensats polysiloxanes-polyuréthanne/polyurée peuvent varier dans de larges limites, en particulier entre 2000 et 500 000, mais de préférence entre 3000 et 250 000.

De préférence, la séquence polysiloxanique répond à la formule générale (I) suivante :

$$-\!\left\{ \left[ X^1 - P - X^1 \right] \underset{\underset{O}{\|}}{C} - NH - R - NH - \underset{\underset{O}{\|}}{C} \right\}\!- \qquad (I)$$

dans laquelle P est un segment polysiloxane, $X^1$ représente, séparément ou conjointement, -O- ou -NH-, et R (qui n'est autre que le motif du diisocyanate tel que mentionné ci-avant) est un radical divalent choisi parmi les radicaux alkylènes de type aromatique, aliphatique ou cycloaliphatique.

De préférence, le segment polysiloxane P répond à la formule générale (I') suivante :

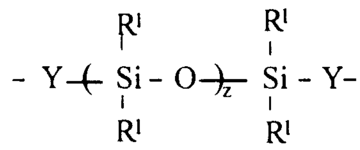 (I')

dans laquelle les radicaux $R^1$, qui peuvent être identiques ou différents, sont choisis parmi d'une part les radicaux hydrocarbonés monovalents en C1-C20 exempts ou substantiellement exempts d'insaturations éthyléniques et, d'autre part, les radicaux aromatiques, Y représente un radical hydrocarboné bivalent, et z un nombre entier tel que le poids moléculaire moyen du segment polysiloxane soit compris entre 300 et 10 000.
De préférence, Y est un radical bivalent choisi parmi les radicaux alkylènes de formule $-(CH_2)_a-$, dans laquelle a représente un nombre entier pouvant être compris entre 1 et 10.
A titre de radicaux $R^1$ convenant dans le cadre de l'invention, on peut plus particulièrement citer les radicaux alkyles, et notamment les radicaux méthyle, éthyle, propyle, isopropyle, butyle, pentyle, hexyle, octyle, décyle, dodécyle et octadécyle, les radicaux cycloalkyles, en particulier le radical cyclohexyle, les radicaux aryles, notamment phényle et naphtyle, les radicaux arylalkyles, notamment benzyle et phényléthyle, ainsi que les radicaux tolyle et xylyle.
On notera que, selon l'invention, il est important que le segment polysiloxane soit exempt, ou substantiellement exempt, de motifs du type Si-H ou Si-$R^1$ dans lequel $R^1$ représenterait un radical hydrocarboné présentant des insaturations éthyléniques, et ceci de manière à éviter toute réticulation intempestive du polycondensat sur lui-même.
Selon un mode particulièrement préféré de réalisation de la présente invention, le segment polysiloxane P présent dans les polycondensats constituant le pseudo-latex, répond à la formule (I") suivante :

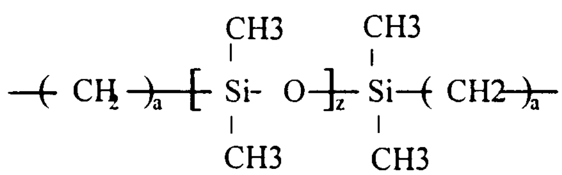 (I")

dans laquelle a et z sont des valeurs telles que définies ci-avant.

Concernant maintenant les séquences polyuréthannes et/ou polyurées rentrant dans la constitution des polycondensats qui sont utilisés dans le cadre de l'invention, celles-ci répondent de préférence à la formule générale (II) suivante :

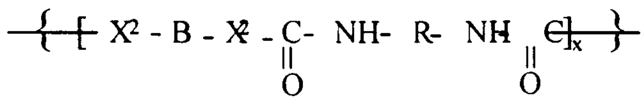 (II)

dans laquelle $X^2$ représente, séparément ou conjointement, -O- ou -NH-; R (qui, comme ci-avant pour la formule (1), est le motif du diisocyanate utilisé pour conduire la réaction de condensation) est tel que défini ci-dessus pour les séquences de formule (I); x (qui, comme indiqué précédemment dans la description correspond substantiellement au nombre de moles d'agents coupleurs utilisées dans le procédé de synthèse du polycondensat) est un nombre entier pouvant varier de 1 à 10, et de préférence de 1 à 3; et B (qui est le motif apporté par l'agent coupleur tel que mentionné ci-avant) est un radical hydrocarboné bivalent porteur d'une charge ionique, positive ou négative.
A titre de radicaux B porteurs de groupements anioniques (i.e de charges négatives), on peut plus particulièrement citer ceux qui sont porteurs de groupement présentant une ou des fonction(s) carboxylique(s) et/ou une ou des fonctions sulfoniques, lesdites fonctions carboxyliques et/ou sulfoniques étant neutralisées partiellement ou totalement par une base minérale ou organique, comme cela sera expliqué plus en détails par la suite.
Ainsi, parmi les radicaux B bivalents porteurs de fonctions carboxyliques ou sulfoniques convenant particulièrement bien dans le cadre de la présente invention, on peut mentionner ceux de formule (III):

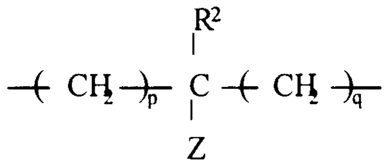 (III)

dans laquelle $R^2$ représente un radical alkyle, linéaire ou ramifié, en C1-C3; Z une fonction acide carboxylique (-COOH) ou acide sulfonique ($-SO_3H$) ou un sel desdites fonctions acides (fonctions carboxylate et sulfonate, respectivement), et p et q, qui peuvent être identiques ou différents, des nombres entiers compris entre 1 et 5,
et ceux de formule (III') :

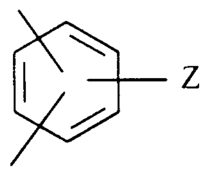 (III')

dans laquelle Z a la signification ci-dessus,
A titre de radicaux B porteurs de groupements cationiques (i.e. de charges positives), on peut plus particulièrement citer ceux qui sont porteurs de groupements de type amines tertiaires, lesdites amines tertiaires étant, pour partie ou totalement, soit neutralisées (présence de motifs $-NH^+-$) soit quaternisées, comme cela sera expliqué plus en détails par la suite.
Ainsi, parmi les radicaux B bivalents porteurs de fonctions amines tertiaires cationisables convenant particulièrement bien dans le cadre de la présente invention, on peut mentionner ceux de formule :

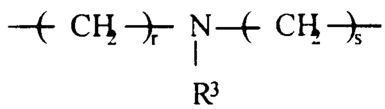 (IV)

dans laquelle $R^3$ représente un radical alkyle, linéaire ou ramifié, en C1-C4, et r et s deux nombres entiers, identiques ou différents, qui peuvent être compris entre 1 et 10.
Sous forme neutralisée ou quaternisée, les radicaux B ci-dessus deviennent alors :

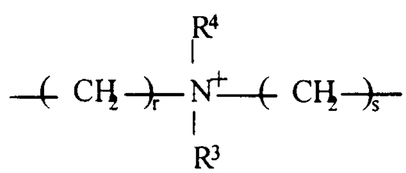 (IV')

formule dans laquelle $R^3$ a la signification ci-dessus, et $R^4$ représente soit l'hydrogène (neutralisation) soit un radical alkyle, linéaire ou ramifié, en C1-C10 ou un cycle aromatique (quaternisation).
Selon l'invention, les taux de neutralisation des fonctions anionisables ou cationisables peuvent être compris entre 10 et 100%, de préférence entre 20 et 100%.
Concernant enfin les radicaux R plus particulièrement préférés selon la présente invention et rentrant dans le cadre de la définition des séquences de formules (I) et (II) données ci-avant, on peut mentionner ceux de formules :

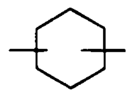 , 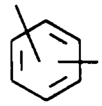 , 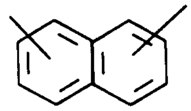

ou

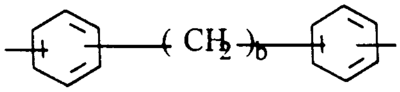 , 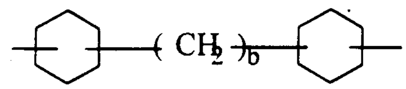 , ou 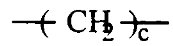

dans lesquelles b est un nombre entier compris entre 0 et 3 , et c un nombre entier compris entre 1 et 20, de préférence compris entre 2 et 12.

Parmi les radicaux bivalents R particulièrement préférés rentrant dans le cadre des formules ci-dessus, on peut citer les radicaux hexaméthylène, 4,4'-biphénylèneméthane, 2,4- et/ou 2,6-tolylène, 1,5-naphtylène, p-phénylène, méthylène 4,4-biscyclohéxyle et le radical divalent dérivé de l'isophorone.

Le procédé de synthèse des pseudo-latex utilisés dans le cadre de la présente invention va maintenant être développé un peu plus en détails.

Dans ses grandes lignes, ce procédé correspond à celui déjà indiqué en début de description.

On fait réagir, dans un solvant organique, un $\alpha,\omega$-dihydroxy et/ou diamino et/ou aminohydroxy et/ou hydroxyaminopolysiloxane répondant à la formule générale $X^3$- P- $X^3$ dans laquelle P a la signification donnée ci-avant (segment polysiloxane), et $X^3$ représente, conjointement ou séparément, -OH ou -$NH_2$, avec un excès stoechiométrique d'un diisocyanate de formule O=C=N-R-N=C=O dans laquelle R a la signification donnée ci-avant, puis on couple les chaînes du polycondensat obtenu précédemment par un diol et/ou une diamine et/ou une alcool amine répondant à la formule

$X^4$- B- $X^4$ dans laquelle B a la signification donnée ci-avant, et $X^4$ représente -OH ou -$NH_2$, à une température comprise entre 40 et 100°C, en présence d'un sel d'étain en tant que catalyseur.

Le solvant organique utilisé à ces étapes est de préférence choisi dans le groupe constitué par l'acétone, la méthyléthylcétone, le tétrahydrofuranne et le 1,2-dichloroéthane, ces solvants étant inertes vis-à-vis des groupes isocyanates.

Le sel d'étain est, quant à lui, de préférence choisi parmi l'éthyl-2 hexanoate d'étain et le dibutyl dilaurate d'étain.

Dans le cadre de la mise en oeuvre du procédé ci-dessus, les diisocyanates particulièrement préférés sont choisis, seuls ou en mélanges, parmi le diphénylméthane 4,4'-diisocyanate et le méthylène 4,4'-bis-dicyclohexyldiisocyanate, et les agents coupleurs particulièrement préférés sont choisis, seuls ou en mélanges, parmi l'acide diméthylol propionique, la N-méthyldiéthanolamine, le 1,3-diaminopropane et l'éthanolamine, étant bien entendu que la possibilité de mélange coupleur acide/coupleur amine est exclue. Le polycondensat polysiloxane-polyuréthane/polyurée ainsi obtenu peut être ensuite éventuellement purifié, par exemple par précipitation dans un solvant non polaire tel que le cyclohexane.

Conformément à l'invention, ce polycondensat, éventuellement purifié, est ensuite utilisé à la préparation d'un pseudo-latex stable qui sera constitué de particules solides dudit polycondensat neutralisé à l'aide d'un agent neutralisant convenable qui peut être soit une base minérale ou organique lorsque le radical B tel que ci-avant défini est porteur de fonctions anionisables telles que par exemple des fonctions acides carboxyliques et/ou sulfoniques, soit un acide minéral ou organique lorsque ledit radical B est porteur de fonctions cationisables telles que par exemple des fonctions amines tertiaires, soit un halogénure d'alkyle en vue de procéder spécifiquement à la quaternisation d'amines tertiaires. Selon l'invention, le taux de neutralisation peut aller de 10% à 100%, de préférence de 20 à 100 %.

A l'inverse d'un procédé conventionnel de préparation de pseudo-latex qui consiste à dissoudre un polymère insoluble dans l'eau, dans un solvant organique, soluble ou partiellement soluble dans l'eau, à introduire dans la solution organique de polymère ainsi obtenue un tensioactif, un mélange de tensioactifs ou un polymère colloïde protecteur ou bien encore un mélange tensioactif(s)/polymère colloïde protecteur, et ceci dans le but d'obtenir une bonne stabilisation des particules, puis à disperser (émulsion) sous agitation la dispersion ainsi obtenue dans de l'eau et à procéder ensuite à l'élimination du solvant organique par évaporation sous vide, ce qui conduit à une suspension aqueuse de particules de polymère enrobées de tensioactif(s) et/ou de polymère colloïde protecteur, les polycondensats à séquences polysiloxane-polyuréthane/polyurée utilisés dans le cadre de l'invention, puisqu'ils comportent des fonctions ioniques partiellement ou totalement neutralisées apportant aux polycondensats une sorte d'"autodispersibilité" dans l'eau, permettent d'obtenir des pseudo-latex particulièrement stables en l'absence de tout stabilisant hydrophile, de tensioactif ou de colloïde protecteur.

Il va de soi que la nature de l'agent neutralisant qu'il convient d'utiliser pour neutraliser le polycondensat polysiloxane-polyuréthane/polyurée est fonction de la nature des fonctions ionisables portées par ce dernier.

Lorsque ledit polycondensat comporte une fonction anionisable telle que par exemple une fonction acide carboxylique

ou sulfonique, l'agent neutralisant peut être une base minérale telle que la soude, la potasse ou l'ammoniaque, ou une base organique telle qu'un aminoalcool choisi notamment parmi le 2-amino 2-méthyl 1-propanol (AMP), la triéthanolamine, la triisopropanolamine (TIPA), la monoéthanolamine, la diéthanolamine, la tri[(2-hydroxy) 1-propyl]amine, le 2-amino 2-méthyl 1,3-propanediol (AMPD), et le 2-amino 2-hydroxyméthyl 1,3-propanediol ou bien une diamine telle que la lysine.
Lorsque le polycondensat comporte une fonction cationisable du type amine tertiaire, l'agent neutralisant peut être un acide minéral tel que l'acide chlorhydrique, ou un acide organique tel que l'acide lactique, l'acide glycolique ou l'acide mandélique. L'agent neutralisant peut être aussi un agent quaternisant de la fonction amine tertiaire, comme par exemple les halogénures d'alkyles et en particulier le iodure de méthyle ou le bromure d'éthyle.
La neutralisation peut être réalisée soit in situ dans la solution du polycondensat polysiloxane-polyuréthane/polyurée dans le solvant organique par addition de la quantité déterminée d'agent neutralisant, soit lors de la préparation de l'émulsion, l'agent neutralisant se trouvant alors dans la phase aqueuse de l'émulsion.

Le solvant organique utilisé doit être un solvant volatil ou un mélange de tels solvants présentant un point d'ébullition inférieur à celui de l'eau et être par ailleurs miscible ou partiellement miscible à l'eau. Un tel solvant organique est de préférence choisi parmi l'acétone, la méthyléthylcétone, le tétrahydrofuranne, l'acétate de méthyle, l'acétate d'éthyle, l'isopropanol et l'éthanol.

Après l'obtention du polycondensat polysiloxane-polyuréthane/polyurée totalement ou partiellement neutralisé dans le solvant organique, on procède alors à la préparation d'une émulsion en versant, sous agitation, à la solution organique obtenue, une quantité appropriée d'eau contenant éventuellement un agent anti-mousse dont le rôle sera de faciliter l'évaporation ultérieure de la phase organique.
Comme indiqué précédemment, on peut, selon une variante du procédé, opérer la neutralisation des fonctions ionisables du polycondensat lors de la formation même de l'émulsion en versant une solution aqueuse contenant la quantité requise de l'agent neutralisant.
Lors de la formation de l'émulsion, l'agitation est de préférence réalisée à l'aide d'un disperseur cisaillant du type Moritz ou Ultraturax ou Raineri équipé de pâles défloculantes.
L'émulsion ainsi obtenue est particulièrement stable sans qu'il soit nécessaire d'employer un agent tensioactif dans la mesure où les groupes ioniques du polycondensat polysiloxane-polyuréthane/polyurée se placent à l'interface avec l'eau et protègent les gouttelettes de la coalescence par répulsion électrostatique. Après formation de l'émulsion à une température comprise entre la température ambiante et 70°C environ, on procède alors à l'évaporation sous pression réduite du solvant organique jusqu'à son élimination totale, ladite évaporation étant de préférence réalisée sous léger chauffage.

On obtient ainsi finalement un pseudo-latex, c'est à dire une dispersion aqueuse de particules du polycondensat polysiloxane-polyuréthane/polyurée filmogène, qui est exempte de tout tensioactif ou de tout autre stabilisant hydrophile, tout en étant très stable.
La taille moyenne des particules constituant le pseudo-latex, ainsi que leur polydispersité, peuvent être réglées en faisant varier, lors de la préparation dudit pseudo-latex, les proportions respectives entre le polycondensat, le solvant organique et l'eau, ou en faisant varier le taux de neutralisation ou la nature de l'agent neutralisant.
Selon un mode particulier de réalisation des pseudo-latex utilisés dans le cadre de la présente invention, la taille moyenne des particules constituant ledit pseudo-latex est comprise entre 5 et 400 nanomètres, de préférence entre 10 et 250 nanomètres.

La polydispersité en taille desdites particules, mesurée par diffusion quasi-élastique de la lumière, est, quant à elle, généralement inférieure à 0,5, et de préférence inférieure à 0,3.

Comme indiqué précédemment, les compositions cosmétiques selon l'invention, qui contiennent donc, dans un support cosmétiquement acceptable, des pseudo-latex tels que ci-dessus définis, présentent, pour des applications aussi variées que celles rencontrées par exemple dans le domaine du capillaire, du maquillage ou bien encore des soins de la peau, ou de tout autre domaine cosmétique dans lequel l'utilisation d'une substance filmogène est désirable ou recherchée, des propriétés tout à fait remarquables, en particulier au niveau de leurs propriétés filmogènes et de brillance, de leur aptitude à conserver ces propriétés dans le temps face à l'action d'agents extérieurs (rémanence) et aussi de leur propriétés de douceur, de lubrification et de résistance à l'abrasion.
Parmi les applications préférentiellement visées par la présente invention, et les différents effets bénéfiques obtenus dans ces dernières, on peut plus particulièrement mentionner :

- le domaine des produits capillaires (lavage, soin ou beauté des cheveux), où les compositions selon l'invention, en particulier sous forme d'aérosols, de mousse, de shampooings, d'après-shampooings, de lotions ou de gels coiffants ou traitants, laques ou lotions de mise en forme ou de mise en plis ou encore de fixation, permettent d'apporter aux cheveux brillance, douceur, facilité de coiffage (phénomène d'"individualisation" des cheveux au moment du dépôt de la composition), meilleur toucher, ainsi que la rémanence (c'est à dire le maintien durable, même sous l'action d'agents extérieurs) de ces propriétés.

- le domaine des produits de maquillage, en particulier pour le maquillage des ongles et des cils, où les compositions selon l'invention, sous forme de vernis à ongle, de mascaras ou de eye-liners par exemple, permettent d'apporter, dans le cas du maquillage des cils, les mêmes avantages que ceux évoqués précédemment pour le traitement des cheveux, et, dans le cas des vernis à ongles (où les compositions peuvent être utilisées comme filmogène seule ou comme additif filmogène), brillance, meilleure mouillabilité de l'ongle, rémanence du film et de sa brillance aux lavages, meilleure résistance à l'abrasion (apport de glissant par lubrification des surfaces), meilleure rigidité.
- le domaine des produits de soin de la peau (crèmes, laits, lotions, masques, sérums, produits solaires), où les compositions selon l'invention permettent plus particulièrement d'apporter brillance, meilleure mouillabilité et résistance aux lavages à l'eau (produits solaires).

La proportion en pseudo-latex dans les compositions cosmétiques (hors vernis à ongles) est généralement comprise entre 0,5 et 20%, et de préférence entre 1 et 15% en poids par rapport au poids total de la composition. Dans le cas des vernis à ongles, cette proportion peut aller jusqu'à 30% en poids.
Les compositions peuvent en outre, et bien entendu, contenir divers adjuvants destinés à la rendre acceptable dans une application cosmétique particulière. Les compositions selon l'invention peuvent contenir des filtres solaires UV-A ou UV-B ou à bande large et être utilisées ainsi comme produits anti-solaires.
Les compositions selon l'invention peuvent par ailleurs contenir des additifs cosmétiques conventionnels choisis parmi les corps gras, des solvants organiques, des silicones, des agents épaississants, des adoucissants, des agents anti-mousse, des agents hydratants, des humectants, des agents traitants (agents anti-chute, antipelliculaire,...), des polymères anioniques, non ioniques ou amphotères ou leurs mélanges, des anti-transpirants, des agents alcanisants, des colorants, des pigments, des parfums, des conservateurs et des agents propulseurs lorsque les compositions se présentent sous forme aérosol.
Plus précisément, comme corps gras, on peut utiliser une huile ou une cire ou leurs mélanges, des acides gras, des alcools gras, des esters d'acides gras tels que les triglycérides d'acides gras en C6-C18, de la vaseline, de la paraffine, de la lanoline, de la lanoline hydrogénée ou acétylée.
Parmi les huiles, on peut citer les huiles minérales, animales, végétales ou les huiles de synthèse et notamment l'huile de vaseline, de paraffine, de ricin, de jojoba, de sésame, ainsi que les huiles et les gommes de silicones et les isoparaffines.
Parmi les cires animales, fossiles, végétales, minérales ou de synthèse, on peut notamment citer la cire d'abeille, de caroube, de Candellila, l'ozokérite, les cires microcristallines ainsi que les cires et les résines de silicone.
Parmi les agents épaississants, on peut citer :

- les celluloses modifiées telles que l'hydroxyéthylcellulose, la méthylcellulose, l'hydroxypropylcellulose et la carboxyméthylcellulose. Parmi celles-ci, on peut citer notamment les gommes vendues sous la dénomination de "Cellosize QP 44001H par la Société Amercol,
- la gomme de caroube, la gomme de guar, la gomme de guar quaternisée vendue sous la dénomination de "Jaguar C-13-S" par la Société Meyhall, la gomme d'hydroxypropylguar, la gomme de xanthane,
- les acides polyacryliques réticulés tels que les "Carbopol" de la Société Goodrich,
- les polymères poly(métha)crylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic, ou de "Salcare SC95" par la Société Allied Colloïd, ou encore
- les homopolymères réticulés de chlorure de méthacryloyloxyéthyltriméthylammonium vendus sous la dénomination de "Salcare SC95" par la Société Allied Colloïd.

On va maintenant donner à titre d'illustration de l'invention plusieurs exemples de préparation de polycondensats polysiloxane-polyuréthane ainsi que de pseudo-latex et de compositions cosmétiques les contenant.

Les synthèses conduisant aux polycondensats multiséquencés polysiloxanepolyuréthane ont été réalisées à partir de prépolymères du type polydiméthylsiloxane $\alpha,\omega$ hydroxyorganofonctionnels (produits commerciaux vendus par la Société Goldschmidt sous les dénominations Tegomer H-Si 2111 et Tegomer H-Si 2311) de structure :

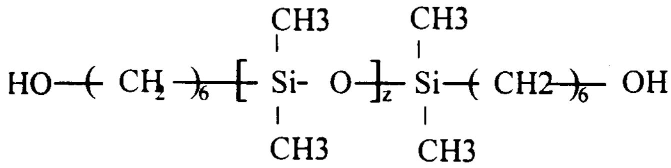

et ayant en outre les caractéristiques rassemblées dans le tableau ci-dessous :

| Nom commercial | Tegomer H-Si 2111 | Tegomer H-Si 2311 |
|---|---|---|
| Groupes fonctionnels | groupes hydroxyles primaires | |
| Fonctionnalité | 2 | 2 |
| nombre de motifs z | environ 10 | environ 30 |
| Indice Hydroxyle (mg KOH/g) | 120 (+/- 10) | 45 (+/- 5) |
| viscosité à 25°C (cP) | 85 (+/- 10) | 115 (+/- 15) |
| Poids moléculaire moyen en nombre (Mn) | 700 | 2 200 |

Dans la suite, ces deux produits commerciaux seront appelés par commodité SIL 700 et SIL 2200 (nomination basée sur leur poids moléculaire respectif).

**Exemple 1**

On prépare un polycondensat polysiloxane-polyuréthane anionisable de structure théorique :

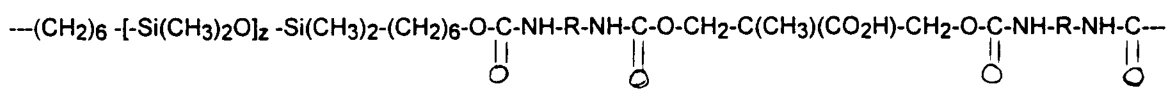

dans laquelle R représente :

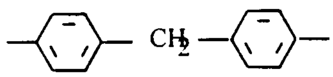

et correspondant à la réaction entre :

- 1 mole de SIL 700 (prépolymère polysiloxane)
- 2 moles de 4,4' diphénylméthanediisocyanate (ci-après dénommé MDI)
- et 1 mole d'acide diméthylolpropionique (agent coupleur, ci-après dénommé DMPA),

ces valeurs étant rapportées à 1 mole de SIL 700.

Dans un réacteur cylindrique pourvu d'une agitation centrale du type ancre, d'un thermomètre, d'un réfrigérant, d'une arrivée de barbotage d'azote et surmonté d'une ampoule à introduction, on introduit, sous courant d'azote, 50 g de MDI et 50 g de tétrahydrofurane (THF). La dissolution du mélange se fait sous agitation et à température ambiante.

Parallèlement, on dissout 70 g de SIL 700 dans 70 g de THF, et la solution ainsi obtenue est versée dans l'ampoule à introduction située au dessus du réacteur.

On introduit alors, sous agitation et courant d'azote, cette solution de SIL 700 dans le réacteur contenant la solution de MDI, en maintenant la température du milieu de réaction à 50°C par chauffage extérieur.

La coulée de la solution de SIL 700 dure 1h30 et la température du milieu réactionnel est maintenue à 50°C pendant toute la durée de l'introduction.

A la fin de la coulée, la réaction conduit à la formation quantitative d'un prépolymère polysiloxane à extrémités $\alpha,\omega$ dii-

socyanate.
On introduit ensuite (temps de coulée : 30 min) dans le réacteur contenant le prépolymère ci-dessus, et ceci toujours sous agitation, barbotage d'azote et maintien de la température à 50°C, une solution de DMPA obtenue en dissolvant 13,4 g de DMPA dans 400 g de THF.
Au début de la coulée, on introduit en outre dans le milieu de réaction 0,15 g de dibutyldilaurate d'étain qui sert de catalyseur. On laisse ensuite réagir le tout pendant 10 h, sous agitation et à 50°C.
La fin de la réaction peut être contrôlée en vérifiant par analyse infrarouge l'absence de bandes d'adsorption -N=C=O à 2270 $cm^{-1}$. Si besoin est, on peut alors rajouter de l'éthanol au milieu réactionnel pour terminer la réaction et consommer totalement les groupes -N=C=O encore disponibles; dans ce cas, on peut par exemple rajouter de l'ordre de 10 ml d'éthanol et laisser à nouveau réagir le tout pendant 4 h à 50°C.
En fin de réaction, on obtient une solution organique (THF) du polycondensat désiré, lequel est ensuite récupéré et purifié par précipitation de ladite solution dans 5 l d'un mélange équi-volume (50/50) éther de pétrole/éther éthylique.
Le rendement de récupération est de 90% en poids après séchage.
L'indice d'acide du polycondensat obtenu est de 46 (théorique : 42).
Son poids moléculaire moyen en nombre est de 5000.

**<u>Exemple 2</u>**

On prépare un polycondensat polysiloxane-polyuréthane anionisable de même structure théorique que celle de l'exemple 1, mais à partir du prépolymère polysiloxane SIL 2200
Le mode opératoire suivi est identique à celui de l'exemple 1, mais les quantités de réactifs mises en jeu sont les suivantes :

- 80 g de SIL 2200 dissous dans 80 g de THF
- 18,2 g de MDI dissous dans 20 g de THF
- 4,9 g de DMPA dissous dans 200 g de THF
- 0,1 g de dibutyldilaurate d'étain

de manière à avoir les proportions 1 mole SIL 2200/2 moles MDI/1 mole DMPA. La récupération et la purification du polycondensat final désiré se font plus simplement par précipitation de la solution organique le contenant dans 5 l d'eau permutée.
Le rendement de récupération est alors de 92% en poids.
L'indice d'acide du polycondensat obtenu est de 21,7 (théorique : 19,8).
Son poids moléculaire moyen en nombre est de 6300.

**<u>Exemple 3</u>**

On prépare un polycondensat polysiloxane-polyuréthane cationisable de structure théorique :

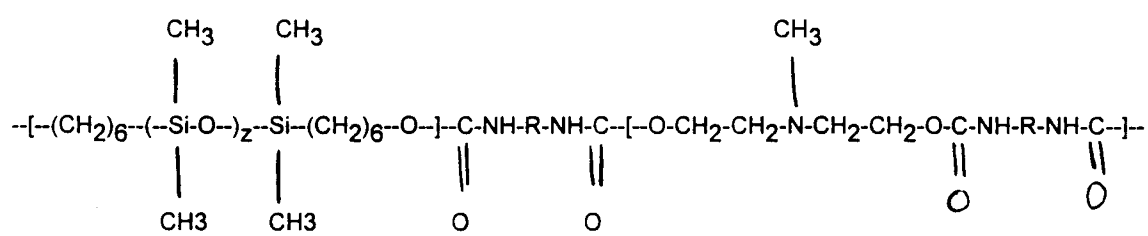

dans laquelle R représente :

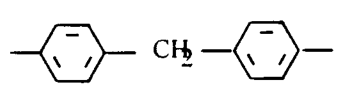

et correspondant à la réaction entre :

- 1 mole de SIL 700
- 2 moles de MDI
- 1 mole de N méthyldiéthanolamine (agent coupleur, ci-après dénommé MEA)

ces valeurs étant rapportées à 1 mole de SIL 700.

Dans le même réacteur que celui de l'exemple 1 et équipé de la même manière, on introduit, sous courant d'azote, 50 g de MDI et 50 g de THF.

La dissolution du mélange se fait sous agitation et à température ambiante.

Parallèlement, on dissout 70 g de SIL 700 dans 70 g de THF, et la solution ainsi obtenue est versée dans l'ampoule à introduction située au dessus du réacteur.

On introduit alors, sous agitation et courant d'azote, cette solution de SIL 700 dans le réacteur contenant la solution de MDI, en maintenant la température du milieu de réaction à 50°C par chauffage extérieur.

La coulée de la solution de SIL 700 dure 1h30 et la température du milieu réactionnel est maintenue à 50°C pendant toute la durée de l'introduction.

A la fin de la coulée, on dilue le milieu de réaction par 350 g de THF, tout en maintenant la température à 50°C.

La réaction conduit à la formation quantitative d'un prépolymère polysiloxane à extrémités $\alpha,\omega$ diisocyanate.

On introduit ensuite (temps de coulée : 30 min) dans le réacteur contenant le prépolymère ci-dessus, et ceci toujours sous agitation, barbotage d'azote et maintien de la température à 50°C, une solution de MEA obtenue en dissolvant 12,5 g de MEA dans 70 g de THF.

On laisse ensuite réagir le tout pendant 7h, sous agitation et à 50°C.

La fin de la réaction peut être contrôlée en vérifiant par analyse infrarouge l'absence de bandes d'adsorption -N=C=O à 2270 $cm^{-1}$. Si besoin est, on peut alors rajouter de l'éthanol au milieu réactionnel pour terminer la réaction et consommer totalement les groupes -N=C=O encore disponibles; dans ce cas, on peut par exemple rajouter de l'ordre de 10 ml d'éthanol et laisser à nouveau réagir le tout pendant 4 h à 50°C.

En fin de réaction, on obtient une solution organique (THF) du polycondensat désiré, lequel est ensuite récupéré et purifié par précipitation de ladite solution dans 5 l d'un mélange équi-volume (50/50) éther de pétrole/éther éthylique.

Le rendement de récupération est de 93% en poids après séchage.

L'indice d'amine du polycondensat obtenu est de 45,7 (théorique : 43).

Son poids moléculaire moyen en nombre est de 12600.

**Exemple 4**

On prépare un polycondensat polysiloxane-polyuréthane cationisable de même structure théorique que celle de l'exemple 3, mais obtenu à partir du prépolymère polysiloxane SIL 2200.

Le mode opératoire suivi est donc identique à celui de l'exemple 3, mais les quantités de réactifs mises en jeu sont les suivantes :

- 80 g de SIL 2200 dissous dans 80 g de THF
- 18,2 g de MDI dissous dans 20 g de THF
- 4,55g de MEA dissous dans 25 g de THF

de manière à avoir les proportions 1 mole SIL 2200/2 moles MDI/1 mole MEA.

En outre, la récupération et la purification du polycondensat final désiré se font par précipitation de la solution organique le contenant dans 5 l d'eau permutée. Le rendement de récupération est alors de 90% en poids.

L'indice d'acide du polycondensat obtenu est de 19 (théorique : 20,7).

Son poids moléculaire moyen en nombre est de 50 100.

**Exemple 5**

On prépare un pseudo-latex anionique à partir du polycondensat polysiloxane-polyuréthane anionisable préparé à l'exemple 1, ledit polycondensat étant ici neutralisé *in situ* à 50% (d'après l'indice d'acide) par de la soude.

On dissout 30 g du polycondensat tel que directement obtenu à l'exemple 1 dans 170 g de THF (solution à 15% en poids de polycondensat).

La solution organique ainsi obtenue est placée dans un réacteur et agitée vigoureusement par un disperseur cisaillant du type ULTRA-TURAX. On introduit alors goutte à goutte dans le réacteur contenant la solution organique agitée une phase aqueuse obtenue par mélange de 53,8 g d'eau permutée et de 6,16 g de NaOH 2M (quantité d'agent neutralisant nécessaire pour neutraliser à 50% les groupes -COOH portés par le polycondensat).

Après introduction totale de la phase aqueuse, on ajoute ensuite, toujours sous agitation, 140 g d'eau permutée de

manière à diluer l'émulsion et réaliser complètement l'inversion de phase.
Puis on agite pendant 15 min supplémentaires.
On évapore ensuite sélectivement le solvant organique de l'émulsion grâce à un évaporateur rotatif sous vide partiel, en prenant soin de ne pas dépasser une température de 50°C, et on concentre enfin la dispersion aqueuse de polycondensat à l'extrait sec final désiré, qui est ici de 25 % en poids.
On obtient ainsi un pseudo-latex stable d'aspect laiteux.
La taille moyenne des particules constituant le pseudo-latex est de 120 nm et leur polydispersité en taille est inférieure à 0,1 (mesures par diffusion quasi-élastique de la lumière sur un appareil COULTER N4 SD commercialisé par la Société COULTRONIX).
Le pH de la dispersion est de 6,6.
Cette dispersion donne, par évaporation sur un support, un film continu et régulier, et ceci sans ajout de plastifiant.

**Exemple 6**

On prépare un pseudo-latex anionique à partir du polycondensat polysiloxane-polyuréthane anionisable préparé à l'exemple 2, ledit polycondensat étant ici neutralisé *in situ* à 50% (d'après l'indice d'acide) par de la soude.
Le mode opératoire est absolument identique à celui indiqué à l'exemple 5, mais les quantités et la nature des réactifs mis en jeu sont cette fois les suivantes :

- solution organique : 30 g du polycondensat obtenu à l'exemple 2, dissous dans 170 g de THF
- phase aqueuse contenant le neutralisant : mélange entre 27 g d'eau permutée et 2,7 g de NaOH 2M
- quantité d'eau ajoutée pour la dilution de l'émulsion : 170 g

On obtient ainsi finalement un pseudo-latex anionique présentant les caractéristiques suivantes :

- extrait sec : 25% en poids
- pH : 7,3
- taille moyenne des particules : 92 nanomètres
- polydispersité : 0,25

Comme pour l'exemple 5, le pseudo-latex obtenu donne, après évaporation sur un support, un film continu et régulier, et ceci sans la présence de plastifiant.

**Exemple 7**

On prépare un pseudo-latex cationique à partir du polycondensat polysiloxane-polyuréthane cationisable préparé à l'exemple 3, ledit polycondensat étant neutralisé *in situ* à 30% (d'après l'indice d'amine) par de l'acide chlorhydrique.

Le mode opératoire est identique à celui indiqué à l'exemple 5, mais les quantités et la nature des réactifs mis en jeu sont les suivantes :

- solution organique : 30 g du polycondensat obtenu à l'exemple 3, dissous dans 170 g de THF
- phase aqueuse contenant le neutralisant : mélange entre 27 g d'eau permutée et 3,4 g de HCl 2M
- quantité d'eau ajoutée pour la dilution de l'émulsion : 170 g

On obtient ainsi finalement un pseudo-latex cationique présentant les caractéristiques suivantes :

- extrait sec : 20% en poids
- pH : 4,7
- taille moyenne des particules : 54 nanomètres
- polydispersité : 0,1

Comme pour l'exemple 5, le pseudo-latex obtenu donne, après évaporation sur un support, un film continu et régulier, et ceci sans la présence de plastifiant.

**Exemple 8**

On prépare un pseudo-latex cationique à partir du polycondensat polysiloxane-polyuréthane cationisable préparé à l'exemple 4, ledit polycondensat étant neutralisé *in situ* à 70% (d'après l'indice d'amine) par de l'acide chlorhydrique.

Le mode opératoire est absolument identique à celui indiqué à l'exemple 5, mais les quantités et la nature des réactifs mis en jeu sont cette fois les suivantes:

- solution organique : 30 g du polycondensat obtenu à l'exemple 4, dissous dans 70 g de THF
- phase aqueuse contenant le neutralisant : mélange entre 40 g d'eau permutée et 3,9 g de HCl 2M
- quantité d'eau ajoutée pour la dilution de l'émulsion : 60 g

On obtient ainsi finalement un pseudo-latex cationique présentant les caractéristiques suivantes :

- extrait sec : 20% en poids
- pH : 4,28
- taille moyenne des particules : 75 nanomètres
- polydispersité : 0,25

Comme pour l'exemple 5, le pseudo-latex obtenu donne, après évaporation sur un support, un film continu et régulier, et ceci sans la présence de plastifiant.

**Exemple 9**

On donne ici un exemple de formulation pour vernis à ongles.

| | |
|---|---|
| - Pseudo-latex de l'exemple 5 | 24,88 g en matière active |
| - Epaississant associatif uréthanne non ionique "SER AD FX 1100" de la Société Servo | 0,3 g |
| - Pigments | 1 g |
| - Eau qsp | 100 g |

Le vernis à ongles obtenu est très résistant à l'eau : le film est intact après 1 heure sous agitation dans l'eau.
La dureté du film obtenu est très satisfaisante et celui-ci adhère correctement à la kératine de l'ongle sans s'écailler. Il n'est pas collant et résiste aux rayures.
Le vernis obtenu selon l'invention s'applique facilement sur l'ongle et présente en outre une très bonne brillance, ainsi qu'une tenue satisfaisante.

**Exemple 10**

Cet exemple illustre une formulation pour mascaras.

*Phase A* :

| | |
|---|---|
| - stéarate de triéthanolamine | 11,8 g |
| - cire d'abeille | 5 g |
| - cire de Carnauba | 3 g |
| - paraffine | 1 g |

*Phase B* :

| | |
|---|---|
| - oxyde de fer noir | 5 g |

*Phase C* :

| | |
|---|---|
| - gomme arabique | 2 g |
| - hydroxyéthylcellulose "Cellosize QP" de la Société Amerchol | 1,2 g |

*Phase D* :

| | |
|---|---|
| - pseudo-latex de l'exemple 6 | 5 g de matière active |
| - conservateur qs | |
| - eau qsp | 100 g |

Ce mascara est obtenu en portant les ingrédients de la Phase A à 85°C, à laquelle on ajoute la Phase B et l'on agite à l'aide d'une turbine.
On porte ensuite l'eau de préparation à ébullition, on ajoute les conservateurs, puis à 85°C les ingrédients de la Phase C.

On ajoute alors la phase aqueuse obtenue (85°C) à la Phase A (85°C) sous agitation à l'aide d'une turbine (émulsification) puis on ajoute enfin, à 30°C, le pseudo-latex de la Phase D et agite à l'aide d'une pale.

**Exemple 11**

Cet exemple illustre une autre composition pour mascaras.
On prépare ce mascara selon le même mode opératoire que celui donné à l'exemple 10, mais avec les constituants suivants :

*Phase A*

| | |
|---|---|
| - stéarate de glycérol | 3 g |
| - mélange d'esters d'acide laurique et de sorbitol et d'acide laurique et de sorbitol oxyéthyléné à 20 moles d'oxyde d'éthylène, vendu sous la dénomination de "Tween 20" par la Société ICI | 3,7 g |
| - monoesters d'acide stéarique et de sorbitane vendu sous la dénomination de "Span 60" par la Société ICI | 5,6 g |
| - cire d'abeille | 6 g |
| - cire de Carnauba | 1,8 g |
| - paraffine | 7,8 g |

*Phase B* :

| | |
|---|---|
| - oxyde de fer noir | 4,5 g |

*Phase C* :

| | |
|---|---|
| - hydroxyéthylcéllulose "Cellosize QP" de la Société Amerchol | 1,5 g |

*Phase D* :

| | |
|---|---|
| - pseudo-latex de l'exemple 7 | 2 g |
| - conservateurs qs | |
| - eau qsp | 100 g |

**Exemple 12**

On donne ici quatre exemples de formulations capillaires.

*Lotion de mise en forme* :

| | |
|---|---|
| - pseudo-latex de l'exemple 5 | 5 g de matière active |
| - parfums, colorants, conservateurs qs | |
| - eau déminéralisée qsp | 100 g |

Cette composition, appliquée sur les cheveux, après un shampooing, apporte un bon maintien de la coiffure et une très bonne brillance aux cheveux.

*Lotion de mise en forme* :

| | |
|---|---|
| - pseudo-latex de l'exemple 6 | 4 g de matière active |
| - parfums, colorants, conservateurs qs | |
| - eau déminéralisée qsp | 100 g |

Cette composition, appliquée sur les cheveux, après un shampooing, apporte un bon maintien de la coiffure et une très bonne brillance aux cheveux.

*Spray de coiffage* :

On prépare un spray de coiffage en flacon pompe en conditionnant, dans un récipient approprié, la composition suivante :

| | |
|---|---|
| - pseudo-latex de l'exemple 7 | 3 g de matière active |
| - parfums, colorants, conservateurs qs | |
| - eau déminéralisée qsp | 100 g |

Le récipient, une fois rempli, est ensuite équipé d'une pompe de pulvérisation.
Cette composition donne une bonne tenue de la coiffure et une très bonne brillance aux cheveux.

*Spray de coiffage* :

On prépare un spray de coiffage en mélangeant :

| | |
|---|---|
| - pseudo-latex de l'exemple 8 | 3 g de matière active |
| - parfums, colorants, conservateurs qs | |
| - eau déminéralisée qsp | 100 g |

la lotion obtenue étant ensuite conditionnée dans un pulvérisateur rechargeable en air comprimé.
Cette composition donne une bonne tenue de la coiffure et une très bonne brillance aux cheveux.

**Revendications**

1. Composition cosmétique, caractérisée par le fait qu'elle comprend, dans un support cosmétiquement acceptable, au moins un pseudo-latex à base d'un polycondensat multiséquencé dont la chaîne est constituée par la répétition d'au moins une séquence polysiloxane et d'au moins une séquence polyuréthanne et/ou polyurée, ladite séquence polyuréthanne et/ou polyurée comportant en outre des groupements anioniques ou cationiques.

2. Composition selon la revendication 1, dans laquelle le poids moléculaire moyen en nombre dudit polycondensat est compris entre 2000 et 500 000, de préférence entre 3000 et 250 000.

3. Composition selon l'une quelconque des revendications précédentes, dans laquelle le rapport en nombre entre les séquences de type polyuréthanne et/ou polyurée et les séquences de type polysiloxane dans le polycondensat est compris entre 1 et 10, de préférence entre 1 et 3.

4. Composition selon l'une quelconque des revendications précédentes, dans laquelle lesdits groupements anioniques sont des fonctions carboxyliques ou des fonctions sulfoniques, totalement ou partiellement neutralisées.

5. Composition selon l'une quelconque des revendications 1 à 3, dans laquelle lesdits groupements cationiques sont des amines tertiaires, totalement ou partiellement neutralisées et/ou quaternisées.

6. Composition selon l'une des revendications 4 ou 5, dans laquelle le taux de neutralisation ou de quaternisation est compris entre 10 et 100%, de préférence entre 20 et 100%.

7. Composition selon l'une quelconque des revendications précédentes, dans laquelle la séquence polysiloxane répond à la formule générale (I) suivante :

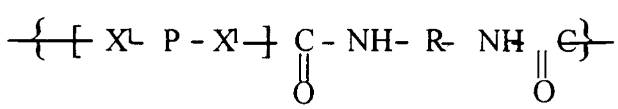
(I)

dans laquelle

- P est un segment polysiloxanique,
- $X^1$ représente, séparément ou conjointement, -O- ou -NH-,
- et R est un radical divalent choisi parmi les radicaux alkylènes de type aromatique, aliphatique ou cycloaliphatique.

8. Composition selon la revendication 7 dans laquelle ledit segment polysiloxanique P répond à la formule générale (I') suivante :

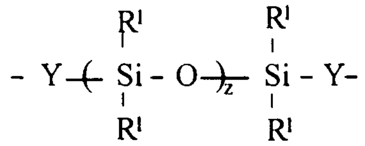 (I')

dans laquelle les radicaux $R^1$, qui peuvent être identiques ou différents, sont choisis parmi d'une part les radicaux hydrocarbonés monovalents en C1-C20 exempts ou substantiellement exempts d'insaturations éthyléniques et, d'autre part, les radicaux aromatiques, Y représente un radical hydrocarboné bivalent, et z un nombre entier tel que le poids moléculaire moyen du segment polysiloxane soit compris entre 300 et 10 000.

**9.** Composition selon la revendication 8, dans laquelle ledit radical bivalent Y est choisi parmi les radicaux alkylènes de formule $-(CH_2)_a-$, dans laquelle a représente un nombre entier pouvant être compris entre 1 et 10.

**10.** Composition selon l'une des revendications 8 ou 9, dans laquelle les radicaux $R^1$ sont choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyles, en particulier le radical cyclohexyle, les radicaux aryles, notamment phényle et naphtyle, les radicaux arylalkyles, notamment benzyle et phényléthyle, ainsi que les radicaux tolyle et xylyle.

**11.** Composition selon la revendication 10, dans laquelle le segment polysiloxane P répond à la formule (I") suivante :

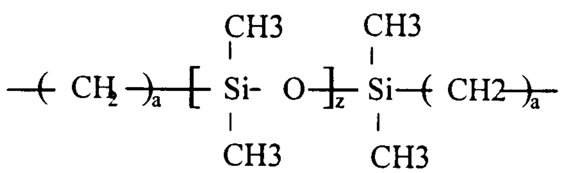 (I")

dans laquelle a et z sont des valeurs telles que définies ci-avant.

**12.** Composition selon l'une quelconque des revendications précédentes, dans laquelle les séquences polyuréthannes et/ou polyurées répondent à la formule générale (II) suivante :

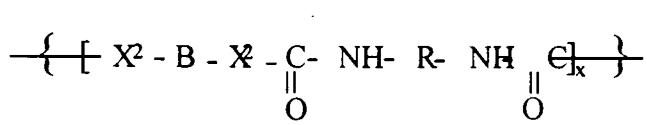 (II)

dans laquelle :

- $X^2$ représente, séparément ou conjointement, -O- ou -NH-,
- R est tel que défini ci-dessus pour les séquences de formule (I),
- x est un nombre entier compris entre 1 à 10, de préférence entre 1 et 3,
- et B est un radical hydrocarboné bivalent porteur d'une charge ionique, positive ou négative.

**13.** Composition selon la revendication 12, dans laquelle le radical B est porteur de groupement présentant une ou des fonction(s) carboxylique(s) et/ou une ou des fonctions sulfoniques, lesdites fonctions carboxyliques et/ou sulfoniques étant neutralisées partiellement ou totalement par une base minérale ou organique.

**14.** Composition selon la revendication 13, dans laquelle le radical B est choisi parmi

- le radical répondant à la formule (III) :

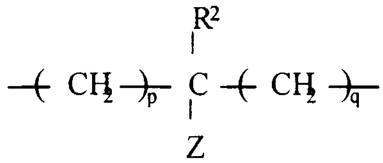 (III)

dans laquelle $R^2$ représente un radical alkyle, linéaire ou ramifié, en C1-C3, Z une fonction acide carboxylique (-COOH) ou acide sulfonique ($-SO_3H$) ou un sel desdites fonctions acides, et p et q, qui peuvent être identiques ou différents, des nombres entiers compris entre 1 et 5, et

- le radical répondant à la formule (III') :

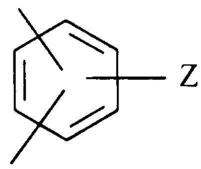 (III')

dans laquelle Z a la signification ci-dessus.

**15.** Composition selon la revendication 12, dans laquelle le radical B est porteur de groupements amines tertiaires, lesdites amines tertiaires étant, partiellement ou totalement, soit neutralisées par une base minérale ou organique, soit quaternisées.

**16.** Composition selon la revendication 15, dans laquelle le radical B répond à la formule (IV) suivante :

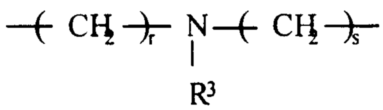 (IV)

dans laquelle $R^3$ représente un radical alkyle, linéaire ou ramifié, en C1-C4, et r et s deux nombres entiers, identiques ou différents, qui peuvent être compris entre 1 et 10.

**17.** Composition selon la revendication 15, dans laquelle, sous forme neutralisée ou quaternisée, le radical B répond à la formule (IV') suivante :

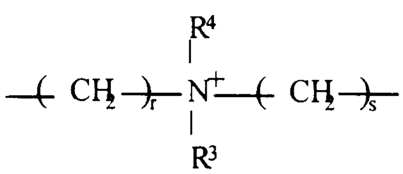 (IV')

dans laquelle $R^3$ a la signification ci-dessus, et $R^4$ représente soit l'hydrogène (neutralisation) soit un radical alkyle, linéaire ou ramifié, en C1-C10 ou un cycle aromatique (quaternisation).

**18.** Composition selon l'une quelconque des revendications 7 à 17, dans laquelle le radical R est choisi parmi les radicaux de formules suivantes :

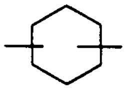 , 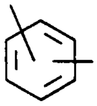 , 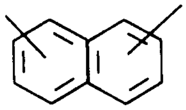

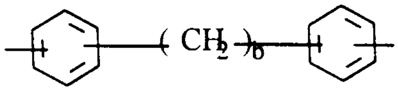

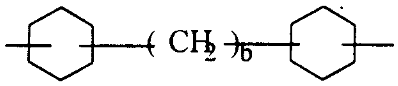

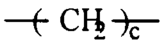

dans lesquelles b est un nombre entier compris entre 0 et 3 , et c un nombre entier compris entre 1 et 20, de préférence compris entre 2 et 12.

**19.** Composition selon la revendication 18, dans laquelle ledit radical R est choisi parmi les radicaux hexaméthylène, 4,4'-biphénylèneméthane, 2,4- et/ou 2,6-tolylène, 1,5-naphtylène, p-phénylène, méthylène 4,4-biscyclohéxyle et le radical divalent dérivé de l'isophorone.

**20.** Composition selon l'une quelconque des revendications précédentes, dans laquelle le polycondensat est obtenu selon un procédé consistant à

- dans une première étape, faire réagir (i) un polymère polysiloxane du type $\alpha,\omega$-dihydroxypolysiloxane ou $\alpha,\omega$-diaminopolysiloxane ou $\alpha,\omega$-aminohydroxy- ou hydroxyamino-polysiloxane) et (ii) un diisocyanate, ledit diisocyanate étant présent en quantité stoechiométrique ou en excès stoechiométrique, ce par quoi l'on obtient un polysiloxane présentant une fonction isocyanate à chacune de ses extrémités de chaîne, puis
- dans une deuxième étape, à coupler les chaînes du polysiloxane obtenu précédemment par réaction avec un agent coupleur choisi parmi les diols et/ou les diamines et/ou les alcoolamines, ledit agent coupleur étant porteur de groupements cationisables ou anioniables, et
- dans une troisième étape, à ioniser, partiellement ou totalement, les groupements cationisables ou anionisables du polycondensat obtenu à l'issue de la deuxième étape.

**21.** Composition selon la revendication 20, dans laquelle le taux de ionisation est compris entre 10 et 100%, de préférence entre 20 et 100%.

**22.** Composition selon l'une des revendications 20 ou 21, dans laquelle le polymère polysiloxane de départ répond à la formule
$X^3$- P- $X^3$ dans laquelle P a la signification donnée ci-avant, et X3 représente, conjointement ou séparément, -OH ou $-NH_2$.

**23.** Composition selon l'une des revendications 20 à 22, dans laquelle le diisocyanate répond à la formule O=C=N-R-N=C=O dans laquelle R a la signification donnée ci-avant.

**24.** Composition selon l'une des revendications 20 à 23 dans laquelle l'agent coupleur répond à la formule :
$X^4$- B- $X^4$ dans laquelle B a la signification donnée ci-avant, et $X^4$ représente -OH ou $-NH_2$.

**25.** Composition selon l'une quelconque des revendications précédentes, dans laquelle la taille moyenne des particules constituant ledit pseudo-latex est comprise entre 5 et 400 nanomètres, de préférence entre 10 et 250 nanomètres.

**26.** Composition selon l'une quelconque des revendications précédentes, dans laquelle la polydispersité en taille des particules constituant ledit pseudo-latex, mesurée par diffusion quasi-élastique de la lumière, est inférieure à 0,5, et de préférence inférieure à 0,3.

**27.** Composition selon l'une quelconque des revendications précédentes, caractérisée en ce qu'il s'agit d'une composition capillaire.

**28.** Composition selon l'une quelconque des revendications 1 à 26, caractérisée en ce qu'il s'agit d'une composition de maquillage.

**29.** Composition selon la revendication 28, caractérisée en ce qu'il s'agit d'un vernis à ongle, et/ou d'un mascara.

**30.** Composition selon l'une quelconque des revendications 1 à 26, caractérisée en ce qu'il s'agit d'une composition pour le soin de la peau.

**31.** Composition selon l'une des revendications précédentes, caractérisée en ce qu'il s'agit d'une composition anti-solaire.

**32.** Utilisation d'un pseudo-latex à base d'un polycondensat multiséquencé tel que défini à l'une quelconque des revendications 1 à 24 comme agent filmogène, ou comme additif d'agent filmogène, dans une composition cosmétique.

**33.** Utilisation selon la revendication 32, dans laquelle ladite composition cosmétique est une composition telle que définie à l'une quelconque des revendications 27 à 31.

**34.** Procédé de traitement non thérapeutique des matières kératiniques, consistant à appliquer sur ces dernières une composition telle que définie à l'une quelconque des revendications 1 à 31.

## Claims

**1.** Cosmetic composition characterized in that it comprises, in a cosmetically acceptable substrate, at least one pseudolatex based on a multiblock polycondensate whose chain consists of the repetition of at least one polysiloxane block and of at least one polyurethane and/or polyurea block, the said polyurethane and/or polyurea block additionally containing anionic or cationic groups.

**2.** Composition according to Claim 1, in which the number-average molecular weight of the said polycondensate is between 2000 and 500 000, preferably between 3000 and 250 000.

**3.** Composition according to any one of the preceding claims, in which the number ratio of the blocks of polyurethane and/or polyurea type to the blocks of polysiloxane type in the polycondensate is between 1 and 10, preferably between 1 and 3.

**4.** Composition according to any one of the preceding claims, in which the said anionic groups are carboxylic functional groups or sulphonic functional groups, completely or partially neutralized.

**5.** Composition according to any one of Claims 1 to 3, in which the said cationic groups are completely or partially neutralized and/or quaternized tertiary amines.

**6.** Composition according to either of Claims 4 and 5, in which the degree of neutralization or of quaternization is between 10 and 100 %, preferably between 20 and 100 %.

**7.** Composition according to any one of the preceding claims, in which the polysiloxane block corresponds to the following general formula (I):

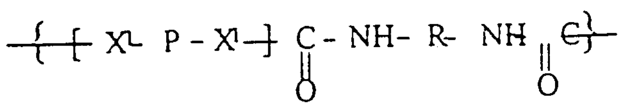 (I)

in which:

- P is a polysiloxane block,
- $X^1$ denotes, separately or jointly, -O- or -NH-, and
- R is a divalent radical chosen from alkylene radicals of aromatic, aliphatic or cycloaliphatic type.

**8.** Composition according to Claim 7, in which the said polysiloxane block P corresponds to the following general formula (I'):

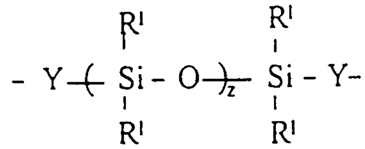 (I')

in which the radicals $R^1$, which may be identical or different, are chosen from, on the one hand, monovalent $C_1$-$C_{20}$ hydrocarbon radicals free from or substantially free from ethylenic unsaturations and, on the other hand, aromatic radicals, Y denotes a divalent hydrocarbon radical and z an integer such that the mean molecular weight of the polysiloxane block is between 300 and 10 000.

**9.** Composition according to Claim 8, in which the said divalent radical Y is chosen from alkylene radicals of formula $-(CH_2)_a-$, in which a denotes an integer which may be between 1 and 10.

**10.** Composition according to either of Claims 8 and 9, in which the radicals $R^1$ are chosen from alkyl radicals, in particular methyl, ethyl, propyl, isopropyl, butyl, pentyl, hexyl, octyl, decyl, dodecyl and octadecyl radicals, cycloalkyl radicals, in particular the cyclohexyl radical, aryl radicals, especially phenyl and naphthyl, arylalkyl radicals, especially benzyl and phenylethyl, and tolyl and xylyl radicals.

**11.** Composition according to Claim 10, in which the polysiloxane block P corresponds to the following formula(I''):

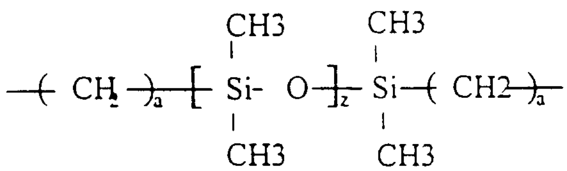 (I'')

in which a and z are values as defined above.

**12.** Composition according to any one of the preceding claims, in which the polyurethane and/or polyurea blocks correspond to the following general formula (II):

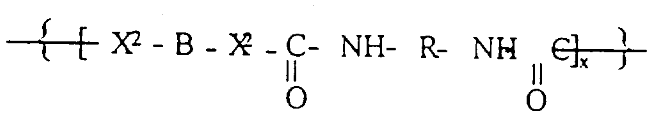 (II)

in which:

- $X^2$ denotes, separately or jointly, -O- or -NH-,
- R is as defined above for the blocks of formula (I),
- x is an integer between 1 and 10, preferably between 1 and 3, and
- B is a divalent hydrocarbon radical carrying a positive or negative ionic charge.

**13.** Composition according to Claim 12, in which the radical B carries a group containing one or more carboxylic functional groups and/or one or more sulphonic functional groups, the said carboxylic and/or sulphonic functional groups being partially or completely neutralized with an inorganic or organic base.

**14.** Composition according to Claim 13, in which the radical B is chosen from

- the radical corresponding to the formula (III):

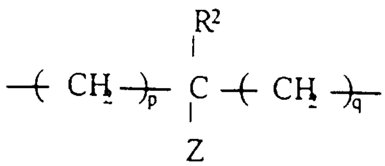 (III)

in which $R^2$ denotes a $C_1$-$C_3$ linear or branched alkyl radical, Z a carboxylic acid (-COOH) or sulphonic acid (-$SO_3H$) functional group or a salt of the said acid functional groups and p and q, which may be identical or different, integers between 1 and 5, and

- the radical B corresponding to the formula (III'):

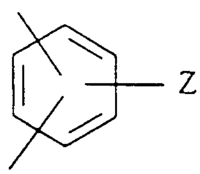 (III')

in which Z has the above meaning.

15. Composition according to Claim 12, in which the radical B carries tertiary amine groups, the said tertiary amines being, partially or completely, either neutralized with an inorganic or organic base or quaternized.

16. Composition according to Claim 15, in which the radical B corresponds to the following formula (IV):

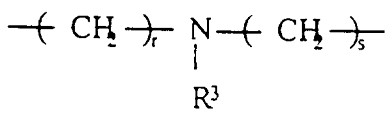 (IV)

in which $R^3$ denotes a $C_1$-$C_4$ linear or branched alkyl radical and r and s two identical or different integers which may be between 1 and 10.

17. Composition according to Claim 15, in which, in neutralized or quaternized form, the radical B corresponds to the following formula (IV'):

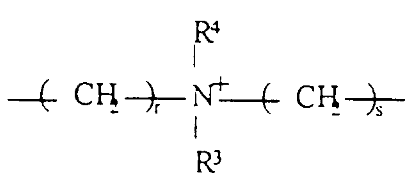 (IV')

in which formula $R^3$ has the above meaning and $R^4$ denotes either hydrogen (neutralization) or a $C_1$-$C_{10}$ linear or branched alkyl radical or an aromatic ring (quaternization).

18. Composition according to any one of Claims 7 to 17, in which the radical R is chosen from the radicals of following formulae:

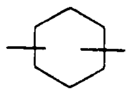,
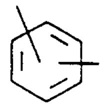,
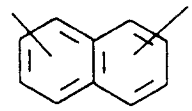

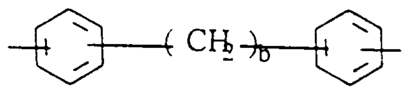

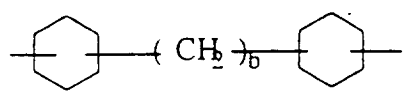

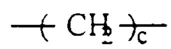

in which b is an integer between 0 and 3 and c an integer between 1 and 20, preferably between 2 and 12.

**19.** Composition according to Claim 18, in which the said radical R is chosen from hexamethylene, 4,4'-biphenylenemethane, 2,4- and/or 2,6-tolylene, 1,5-naphthylene, p-phenylene and 4,4-methylenebiscyclohexyl radicals and the divalent radical derived from isophorone.

**20.** Composition according to any one of the preceding claims, in which the polycondensate is obtained by a process consisting in

- in a first stage, reacting (i) a polysiloxane polymer of the α,ω-dihydroxypolysiloxane or α,ω-diaminopolysiloxane or α,ω-aminohydroxy- or hydroxyaminopolysiloxane type and (ii) a diisocyanate, the said diisocyanate being present in stoichiometric quantity or in stoichiometric excess, whereby a polysiloxane is obtained containing an isocyanate functional group at each of its chain ends, then
- in a second stage, coupling the chains of the polysiloxane obtained previously by reaction with a coupling agent chosen from diols and/or diamines and/or alcoholamines, the said coupling agent carrying cationizable or anionizable groups, and
- in a third stage, partially or completely ionizing the cationizable or anionizable groups of the polycondensate obtained at the end of the second stage.

**21.** Composition according to Claim 20, in which the degree of ionization is between 10 and 100 %, preferably between 20 and 100 %.

**22.** Composition according to either of Claims 20 and 21, in which the starting polysiloxane polymer corresponds to the formula $X^3$- P- $X^3$ in which P has the meaning given above and $X^3$ denotes, jointly or separately, -OH or $-NH_2$.

**23.** Composition according to one of Claims 20 to 22, in which the diisocyanate corresponds to the formula O=C=N-R-N=C=O in which R has the meaning given above.

**24.** Composition according to one of Claims 20 to 23, in which the coupling agent corresponds to the formula $X^4$- B- $X^4$ in which B has the meaning given above and $X^4$ denotes -OH or $-NH_2$.

**25.** Composition according to any one of the preceding claims, in which the mean size of the particles of which the said pseudolatex consists is between 5 and 400 nanometers, preferably between 10 and 250 nanometers.

**26.** Composition according to any one of the preceding claims, in which the size polydispersity of the particles of which the said pseudolatex consists, measured by quasielastic light scattering, is lower than 0.5 and preferably lower than

0.3.

27. Composition according to any one of the preceding claims, characterized in that it is a hair-care composition.

28. Composition according to any one of Claims 1 to 26, characterized in that it is a make-up composition.

29. Composition according to Claim 28, characterized in that it is a nail varnish, and/or a mascara.

30. Composition according to any one of Claims 1 to 26, characterized in that it is a composition for skin care.

31. Composition according to one of the preceding claims, characterized in that it is an antisun composition.

32. Use of a pseudolatex based on a multiblock polycondensate as defined in any one of Claims 1 to 24, as film-forming agent or as film-forming agent additive in a cosmetic composition.

33. Use according to Claim 32, in which the said cosmetic composition is a composition as defined in any one of Claims 27 to 31.

34. Process for the nontherapeutic treatment of keratinous matter, consisting in applying to the latter a composition as defined in any one of Claims 1 to 31.

**Patentansprüche**

1. Kosmetische Zusammensetzung, dadurch gekennzeichnet, daß sie in einem kosmetisch akzeptablen Träger mindestens einen Pseudo-Latex auf der Basis eines Multisequenz-Polykondensats enthält, dessen Kette aus der Wiederholung mindestens einer Polysiloxan-Sequenz und mindestens einer Polyurethan- und/oder Polyharnstoff-Sequenz besteht, wobei die Polyurethan- und/oder Polyharnstoff-Sequenz außerdem anionische oder kationische Gruppen aufweist.

2. Zusammensetzung nach Anspruch 1, wobei das Zahlenmittel des Molekulargewichts des Polykondensats 2 000 bis 500 000, vorzugsweise 3 000 bis 250 000, beträgt.

3. Zusammensetzung nach einem der vorhergehenden Ansprüche, wobei das Zahlenverhältnis zwischen den Sequenzen vom Polyurethan- und/oder Polyharnstofftyp und den Sequenzen vom Polysiloxan-Typ in dem Polykondensat 1 bis 10, vorzugsweise 1 bis 3, beträgt.

4. Zusammensetzung nach einem der vorhergehenden Ansprüche, wobei die anionischen Gruppen Carboxygruppen oder Sulfonsäuregruppen sind, die vollständig oder teilweise neutralisiert sind.

5. Zusammensetzung nach einem der Ansprüche 1 bis 3, wobei die kationischen Gruppen tertiäre Amine sind, die vollständig oder teilweise neutralisiert und/oder quaternisiert sind.

6. Zusammensetzung nach einem der Ansprüche 4 und 5, wobei der Neutralisations- oder Quaternisierungsgrad 10 bis 100 %, vorzugsweise 20 bis 100 %, beträgt.

7. Zusammensetzung nach einem der vorhergehenden Ansprüche, wobei die Polysiloxan-Sequenz der folgenden allgemeinen Formel (I)

$$-\left\{\left[X^1-P-X^1\right]-\underset{\underset{O}{\|}}{C}-NH-R-NH-\underset{\underset{O}{\|}}{C}\right\}- \qquad (I)$$

entspricht, in der bedeuten:

- P ein Polysiloxan-Segment,
- die Reste $X^1$, einzeln oder gemeinsam, -O- oder -NH- und

- R einen zweiwertigen Rest, der unter aromatischen, aliphatischen und cycloaliphatischen Alkylenresten ausgewählt ist.

**8.** Zusammensetzung nach Anspruch 7, wobei das Polysiloxan-Segment P der folgenden allgemeinen Formel (I')

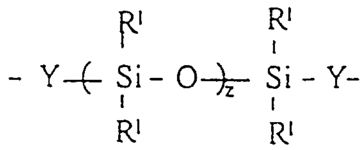

(I')

entspricht, in der die Reste $R^1$, die gleich oder verschieden sein können, einerseits unter einwertigen $C_{1-20}$-Kohlenwasserstoffresten, die vollständig oder im wesentlichen frei von ethylenisch ungesättigten Gruppen sind, und andererseits unter aromatischen Resten ausgewählt sind, in der Y einen zweiwertigen Kohlenwasserstoffrest darstellt und Z eine solche ganze Zahl bedeutet, daß das mittlere Molekulargewicht des Polysiloxan-Segments 300 bis 10 000 beträgt.

**9.** Zusammensetzung nach Anspruch 8, wobei der zweiwertige Rest Y unter Alkylenresten der Formel $-(CH_2)_a-$ ausgewählt ist, in der a eine ganze Zahl von 1 bis 10 darstellt.

**10.** Zusammensetzung nach einem der Ansprüche 8 und 9, wobei die Reste $R^1$ ausgewählt sind unter Alkylresten, insbesondere Methyl, Ethyl, Propyl, Isopropyl, Butyl, Pentyl, Hexyl, Octyl, Decyl, Dodecyl und Octadecyl, Cycloalkylresten, insbesondere Cyclohexyl, Arylresten, insbesondere Phenyl und Naphtyl, Arylalkylresten, insbesondere Benzyl und Phenylethyl, sowie Tolyl und Xylyl.

**11.** Zusammensetzung nach Anspruch 10, wobei das Polysiloxan-Segment P der folgenden Formel (I")

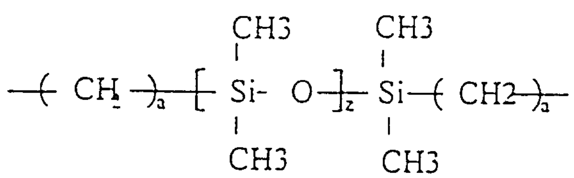

(I")

entspricht, in der a und z die weiter oben definierten Werte darstellen.

**12.** Zusammensetzung nach einem der vorhergehenden Ansprüche, wobei die Polyurethan- und/oder Polyharnstoff-Sequenzen der folgenden allgemeinen Formel (II)

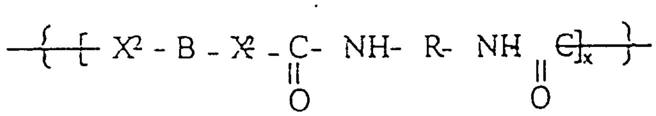

(II)

entsprechen, in der bedeuten:

- die Reste $X^2$, einzeln oder gemeinsam, -O- oder -NH-
- R einen Rest, der wie oben für die Sequenzen der Formel (I) definiert ist,
- x eine ganze Zahl von 1 bis 10 und vorzugsweise 1 bis 3, und
- B einen zweiwertigen Kohlenwasserstoffrest, der eine ionische, positive oder negative Ladung trägt.

**13.** Zusammensetzung nach Anspruch 12, wobei der Rest B eine Gruppe trägt, die eine oder mehrere Carboxygruppen und/oder eine oder mehrere Sulfonsäuregruppen aufweist, die teilweise oder vollständig mit einer anorganischen oder organischen Base neutralisiert sind.

**14.** Zusammensetzung nach Anspruch 13, wobei der Rest B ausgewählt ist unter

- dem Rest der Formel (III)

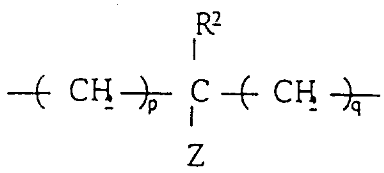

(III),

in der $R^2$ einen geradkettigen oder verzweigten $C_{1-3}$-Alkylrest darstellt, z eine Carboxygruppe (-COOH) oder eine Sulfonsäuregruppe ($-SO_3H$) oder ein Salz dieser Säuregruppen darstellt und p und q, die gleich oder verschieden sein können, ganze Zahlen von 1 bis 5 bedeuten, und
- dem Rest der Formel (III')

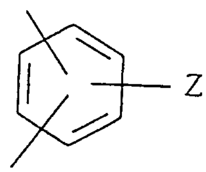

(III'),

in der Z die obige Bedeutung hat.

**15.** Zusammensetzung nach Anspruch 12, wobei der Rest B tertiäre Amingruppen trägt, die teilweise oder vollständig mit einer anorganischen oder organischen Base neutralisiert oder quaternisiert sind.

**16.** Zusammensetzung nach Anspruch 15, wobei der Rest B der folgenden Formel (IV)

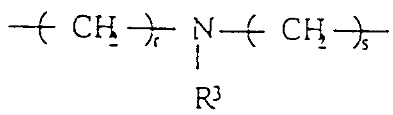

(IV)

entspricht, in der $R^3$ einen geradkettigen oder verzweigten $C_{1-4}$-Alkylrest darstellt und r und s ganze Zahlen bedeuten, die gleich oder verschieden sind und die im Bereich von 1 bis 10 liegen.

**17.** Zusammensetzung nach Anspruch 15, wobei der Rest B, in neutralisierter oder quaternisierter Form, der folgenden Formel (IV')

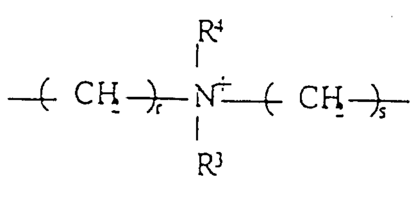

(IV')

entspricht, in der $R^3$ die obige Bedeutung hat und $R^4$ Wasserstoff (Neutralisation) oder einen geradkettigen oder verzweigten $C_{1-10}$-Alkylrest oder einen aromatischen Ring (Quaternisierung) darstellt.

**18.** Zusammensetzung nach einem der Ansprüche 7 bis 17, wobei der Rest R ausgewählt ist unter den Resten der folgenden Formeln:

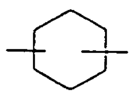 , 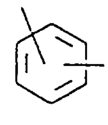 , 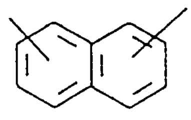

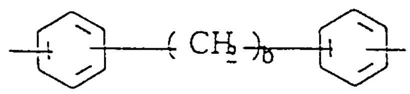

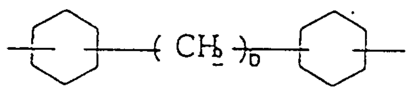

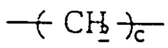

,

in denen b eine ganze Zahl von 0 bis 3 ist und c eine ganze Zahl von 1 bis 20, vorzugsweise 2 bis 12, darstellt.

**19.** Zusammensetzung nach Anspruch 18, wobei der Rest R unter Hexamethylen, 4-4'-Biphenylenmethan, 2,4- und/oder 2,6-Tolylen, 1,5-Naphtylen, p-Phenylen, Methylen-4,4-biscyclohexyl und dem von Isophoron abgeleiteten zweiwertigen Rest ausgewählt ist.

**20.** Zusammensetzung nach einem der vorhergehenden Ansprüche, wobei das Polykondensat nach einem Verfahren hergestellt wird, das darin besteht,

- in einer ersten Stufe (i) ein Polysiloxan-Polymer vom α,ω-Dihydroxypolysiloxan-Typ oder α,ω-Diaminopolysiloxan-Typ oder α,ω-Aminohydroxy- oder - Hydroxyamino-Polysiloxan-Typ mit (ii) einem Diisocyanat umzusetzen, wobei das Diisocyanat in stöchiometrischer Menge oder in stöchiometrischem Überschuß enthalten ist, wodurch ein Polysiloxan erhalten wird, das eine Isocyanat-Gruppe an seinen beiden Kettenenden aufweist, anschließend
- in einer zweiten Stufe die Ketten des zuvor erhaltenen Polysiloxans mit einem Kupplungsmittel, das unter Diolen und/oder Diaminen und/oder Aminoalkoholen ausgewählt wird, zu verbinden, wobei das Kupplungsmittel kationisierbare oder anionisierbare Gruppen trägt, und
- in einer dritten Stufe die kationisierbaren oder anionisierbaren Gruppen des am Ende der zweiten Stufe erhaltenen Polykondensats teilweise oder vollständig zu ionisieren.

**21.** Zusammensetzung nach Anspruch 20, wobei der Ionisierungsgrad 10 bis 100 % und vorzugsweise 20 bis 100 % beträgt.

**22.** Zusammensetzung nach einem der Ansprüche 20 und 21, wobei das als Ausgangsprodukt verwendete Polysiloxan-Polymer der Formel

$$X^3\text{-}P\text{-}X^3$$

entspricht, in der P die weiter oben angegebene Bedeutung hat und die Reste $X^3$, gemeinsam oder einzeln -OH oder $-NH_2$ bedeuten.

**23.** Zusammensetzung nach einem der Ansprüche 20 bis 22, wobei das Diisocyanat der Formel O=C=N-R-N=C=O entspricht, in der R die oben angegebene Bedeutung hat.

**24.** Zusammensetzung nach einem der Ansprüche 20 bis 23, wobei das Kupplungsmittel der Formel

$$X^4\text{-}B\text{-}X^4$$

entspricht, in der B die oben angegebene Bedeutung hat und $X^4$ -OH oder $-NH_2$ darstellt.

**25.** Zusammensetzung nach einem der vorhergehenden Ansprüche, wobei die mittlere Größe der Partikel, die den

Pseudo-Latex bilden, 5 bis 400 nm und vorzugsweise 10 bis 250 nm beträgt.

**26.** Zusammensetzung nach einem der vorhergehenden Ansprüche, wobei die Polydispersität hinsichtlich der Größe der Partikel, aus denen der Pseudo-Latex besteht, gemessen durch quasielastische Lichtstreuung, kleiner als 0,5 und vorzugsweise kleiner als 0,3 ist.

**27.** Zusammensetzung nach einem der vorhergehenden Ansprüche, dadurch gekennzeichnet, daß es sich um eine Zusammensetzung für die Haarbehandlung handelt.

**28.** Zusammensetzung nach einem der Ansprüche 1 bis 26, dadurch gekennzeichnet, daß es sich um eine Schminkzusammensetzung handelt.

**29.** Zusammensetzung nach Anspruch 28, dadurch gekennzeichnet, daß es sich um einen Nagellack und/oder ein Mascara handelt.

**30.** Zusammensetzung nach einem der Ansprüche 1 bis 26, dadurch gekennzeichnet, daß es sich um eine Zusammensetzung für die Hautpflege handelt.

**31.** Zusammensetzung nach einem der vorhergehenden Ansprüche, dadurch gekennzeichnet, daß es sich um eine Lichtschutzzusammensetzung handelt.

**32.** Verwendung eines Pseudo-Latex auf der Basis eines Multisequenz-Polykondensats nach einem der Ansprüche 1 bis 24 als Filmbildner oder als Zusatz zu einem Filmbildner in einer kosmetischen Zusammensetzung.

**33.** Verwendung nach Anspruch 32, wobei die kosmetische Zusammensetzung eine wie in einem der Ansprüche 27 bis 31 definierte Zusammensetzung ist.

**34.** Verfahren zur nicht-therapeutischen Behandlung von Keratinmaterialien, das darin besteht, auf die Keratinmaterialien eine wie in einem der Ansprüche 1 bis 31 definierten Zusammensetzung aufzutragen.